# EUROPÄISCHE PATENTSCHRIFT

(11) **EP 2 077 280 B1**
(45) Veröffentlichungstag und Bekanntmachung des Hinweises auf die Patenterteilung: **24.06.2015**
(21) Anmeldenummer: 09005276.2
(22) Anmeldetag: 19.05.2003
(51) Int. Cl.: C07K 16/18, G01N 33/536, G01N 33/98

(54) **Carbohydrate Deficient Transferrin (CDT)-spezifische Antikörper, ihre Herstellung und Verwendung**
Anti-Carbohydrate Deficient Transferrin (CDT) specific antibodies, their production and use
Anticorps specifiques pour la transferrine déficiente en carbohydrate, leur production et utilisation

(30) Priorität: 05.07.2002 DE 10230550
(43) Veröffentlichungstag der Anmeldung: 08.07.2009
(62) Teilanmeldung aus: 03011334.4
(73) Patentinhaber: Siemens Healthcare Diagnostics Products GmbH, 35041 Marburg (DE)
(72) Erfinder: Althaus, Harald, 35083 Wetter (DE)

(56) Entgegenhaltungen:
- WO-A-00/36418
- WO-A-93/06133
- WO-A-99/00672
- TRIMBLE ESTHER R ET AL: "Anti-peptide antibodies to epitopes masked by the carbohydrate moieties in transferrin." BIOCHEMICAL SOCIETY TRANSACTIONS, Bd. 26, Nr. 1, Februar 1998 (1998-02), Seite S48, XP008022933 663rd Meeting of the Biochemical Society;Galway, Ireland; September 3-5, 1997 ISSN: 0300-5127
- ALTHAUS H ET AL: "Development and evaluation of a new carbohydrate-deficient transferrin (CDT)-specific monoclonal antibody." CLINICAL CHEMISTRY, Bd. 49, Nr. S6, 20. Juni 2003 (2003-06-20) , Seite A113, XP008022934 55th Annual Meeting of the AACC;Philadelphia, PA, USA; July 20-24, 2003 ISSN: 0009-9147
- DELANGHE JORIS R ET AL: "Development and multicenter evaluation of the N Latex CDT direct immunonephelometric assay for serum carbohydrate-deficient transferrin", CLINICAL CHEMISTRY, vol. 53, no. 6, June 2007 (2007-06), pages 1115-1121, ISSN: 0009-9147
- VAN HAERINGEN BART ET AL: "Dynamic structure of human serum transferrin from transient electric birefringence experiments", PROTEINS STRUCTURE FUNCTION AND GENETICS, vol. 23, no. 2, 1995, pages 233-240, ISSN: 0887-3585

### SEQUENCE LISTING

<110> siemens Healthcare Diagnostics Products GmbH
<120> Carbohydrate Deficient Transferrin (CDT) - spezifische Antikörper, ihre Herstellung und verwendung
<130> 2002P59001EP 01
<150> 0301134.4 <151> 2003-05-19
<160> 4
<170> PatentIn version 3.3
<210> 1
   <211> 17
   <212> PRT
   <213> Homo sapiens
<400> 1
   Val Val Ala Arg Ser Met Gly Gly Lys Glu Asp Leu Ile Trp Glu Leu
   1               5                   10                  15
   Leu
<210> 2
   <211> 23
   <212> PRT
   <213> Homo sapiens
<400> 2
   Thr Thr Glu Asp Ser Ile Ala Lys Ile Met Asn Gly Glu Ala Asp Ala
   1               5                   10                  15
   Met Ser Leu Asp Gly Gly Phe
               20
<210> 3
   <211> 15
   <212> PRT
   <213> Homo sapiens
<400> 3
   Ser Lys Leu Ser Met Gly Ser Gly Leu Asn Leu Ser Glu Pro Asn
   1               5                   10                  15
<210> 4
   <211> 13
   <212> PRT
   <213> Homo sapiens
<400> 4
   Tyr Glu Lys Tyr Leu Gly Glu Glu Tyr Val Lys Ala Val
   1               5                   10

## Beschreibung

Die vorliegende Erfindung betrifft monoklonale Antikörper, die in wäßriger Lösung selektiv an ein Transferrin-Homologes "Carbohydrate Deficient Transferrin" (CDT) binden, ohne daß dieses an eine feste Phase gebunden zu sein braucht. CDT ist dadurch gekennzeichnet, daß mindestens eine der zwei Oligosaccharidketten, die normalerweise an Asn 413 und/oder Asn 611 des Transferrins gebunden sind, ganz oder im wesentlichen ganz fehlen.

Alkoholismus ist ein weltweit verbreitetes Problem. Es wurden in der Vergangenheit eine Reihe von diagnostischen Testen entwickelt, um Alkoholismus zu diagnostizieren. Die meisten dieser Teste sind jedoch nicht spezifisch für die Erkrankung. Der bisher am weitesten entwickelte Test wurde von Makhlouf et al. in der EP-0 605 627 vorgestellt. Die darin offenbarten Antikörper reagieren spezifisch mit CDT, das bei Alkoholikern, nicht jedoch bei Nicht-Alkoholikern gefunden wurde. Damit wurde es möglich, einen Immuntest aufzubauen, mit dessen Hilfe CDT in Alkoholiker-Seren nachgewiesen werden kann. Nachteilig ist bei diesem Test jedoch, daß das nachzuweisende Antigen zunächst an eine Festphase gekoppelt werden muß, da die in der EP-0 605 627 offenbarten Antikörper nicht oder nur ungenügend an CDT binden, welches sich in Lösung befindet.

Es bestand somit die Aufgabe, den CDT-Nachweis in der Weise zu verbessern, daß der Direktnachweis von in Lösung befindlichem CDT in einer Probe möglich wird und somit die Notwendigkeit der Koppelung des nachzuweisenden Antigens an eine feste Phase entfällt.

Diese Aufgabe wurde überraschenderweise durch Bereitstellung von Antikörpern gelöst, die in wäßriger Lösung selektiv an CDT binden, ohne daß dieses an eine feste Phase gebunden zu sein braucht. Mit Hilfe von Epitop-Kartierungsexperimenten wurde festgestellt, daß erfindungsgemäße Antikörper, im Gegensatz zu Antikörpern aus dem Stand der Technik, an unterschiedliche Sequenzabschnitte des CDT gleichzeitig binden. Daraus wurde abgeleitet, daß es sich bei den von erfindungsgemäßen Antikörpern erkannten Epitopen um diskontinuierliche Epitope handelt.

Die vorliegende Erfindung betrifft somit einen monoklonalen Antikörper, der in wäßriger Lösung selektiv an CDT bindet, ohne daß dieses an eine feste Phase gebunden zu sein braucht. Es wurde festgestellt, daß die Bindung dieses Antikörpers im Bereich der folgenden Sequenzabschnitte (1) bis (4) des CDT erfolgt:
(1) WARSMGGKEDLIWELL und
(2) TTEDSIAKIMNGEADAMSLDGGF und
(3) SKLSMGSGLNLSEPN und
(4) YEKYLGEEYVKAV,
wobei es unerheblich ist, ob die Peptide festphasengebunden oder in Lösung vorliegen.

Selektive Bindung bedeutet im Rahmen der vorliegenden Erfindung eine ausreichend spezifische oder im wesentlichen spezifische Bindung, die eine deutliche Unterscheidung zwischen CDT einerseits und Humantransferrin andererseits ermöglicht.

Der Begriff "Festphase" oder "feste Phase" im Sinne der vorliegenden Erfindung beinhaltet einen Gegenstand, der aus porösem und/oder nicht porösem, in der Regel wasserunlöslichem Material besteht und die unterschiedlichsten Formen aufweisen kann, wie beispielsweise Gefäß, Röhrchen, Mikrotitrationsplatte, Kugel, Mikropartikel, Stäbchen, Streifen, Filter- oder Chromatographiepapier, etc. In der Regel ist die Oberfläche der Festphase hydrophil oder kann hydrophil gemacht werden. Die Festphase kann aus den unterschiedlichsten Materialien bestehen wie beispielsweise aus anorganischen und/oder organischen Materialien, aus synthetischen, aus natürlich vorkommenden und/oder aus modifizierten natürlich vorkommenden Materialien. Beispiele für Festphasenmaterialien sind Polymere wie beispielsweise Zellulose, Nitrozellulose, Zelluloseacetat, Polyvinylchlorid, Polyacrylamid, vernetzte Dextranmoleküle, Agarose, Polystyrol, Polyethylen, Polypropylen, Polymethacrylat oder Nylon; Keramik; Glas; Metalle, insbesondere Edelmetalle wie Gold oder Silber; Magnetit; Mischungen oder Kombinationen derselben; etc. Auch Zellen, Liposomen oder Phospholipidvesikel sollen vom Begriff "Festphase" mitumfaßt sein.

Die Festphase kann einen Überzug aus einer oder mehreren Schichten aufweisen, beispielsweise aus Proteinen, Kohlehydraten, lipophilen Substanzen, Biopolymeren, organischen Polymeren oder Mischungen hiervon, um beispielsweise die unspezifische Bindung von Probenbestandteilen an die Festphase zu unterdrücken oder zu verhindern oder um beispielsweise Verbesserungen zu erreichen hinsichtlich der Suspensionsstabilität von partikulären Festphasen, der Lagerstabilität, der formgebenden Stabilität oder der Resistenz gegen UV-Licht, Mikroben oder sonstige zerstörend wirkende Agenzien.

Bevorzugt ist der monoklonale Antikörper, der von einer Zellkultur produziert wird, welche bei der DSZM Deutsche Sammlung von Mikroorganismen und Zellkulturen GmbH, Mascheroder Weg 1b, 38124 Braunschweig, Deutschland am 16. April 2002 (Tag des Eingangs bei der Hinterlegungsstelle) nach Budapester Vertrag wie folgt hinterlegt wurde:
Zellkultur 98-84/011 Eingangsnummer: DSM ACC2540

Erfindungsgemäß sind auch antigenbindende Fragmente, beispielsweise Fab- , Fab'-Fv- oder F(ab')₂-Fragmente, die aus den vorstehend genannten erfindungsgemäßen Antikörpern nach den jedem Fachmann bekannten Verfahren hergestellt werden können.

Generell sind unter dem Begriff "Antikörper" im Sinne dieser Erfindung nicht nur komplette Antikörper zu verstehen sondern ausdrücklich auch Antikörperfragmente, wie die bereits genannten Fab-, Fv-, F(ab')₂ oder Fab'-Fragmente sowie auch chimäre, humanisierte, bi- oder oligospezifische, oder "single chain" Antikörper; des weiteren auch Aggregate, Polymere und Konjugate von Immunglobulinen und/oder deren Fragmenten, sofern die Bindungseigenschaften an das Antigen oder Hapten erhalten sind. Antikörperfragmente lassen sich beispielsweise durch enzymatische Spaltung von Antikörpern mit Enzymen wie Pepsin oder Papain herstellen. Antikörperaggregate, -polymere und -konjugate können durch vielfältige Methoden generiert werden, z.B. durch Hitzebehandlung, Umsetzung mit Substanzen wie Glutaraldehyd, Reaktion mit immunglobulinbindenden Molekülen, Biotinylierung von Antikörpern und anschließende Reaktion mit Streptavidin oder Avidin, etc..

Bei einem Antikörper im Sinne dieser Erfindung handelt es sich um einen monoklonalen Antikörper. Der Antikörper kann nach den üblichen Verfahren hergestellt worden sein, z.B. durch Immunisierung eines Tieres, wie z.B. Maus, Ratte, Meerschweichen, Kaninchen, Pferd, Schaf, Ziege, Huhn (s.a. Messerschmid (1996) BIOforum, 11:500-502), und anschließender Etablierung von Hybridomazellen und der anschließenden Reinigung der sekretierten Antikörper; oder durch Klonierung und Expression der Nukleotidsequenzen bzw. modifizierter Versionen davon, welche die Aminosäuresequenzen kodieren, die für die Bindung des natürlichen Antikörpers an das Antigen und/oder Hapten verantwortlich sind.

Weiterhin ist ein Gegenstand der vorliegenden Erfindung ein Verfahren zur Herstellung des Antikörpers durch Immunisieren eines geeigneten Versuchstieres mit nichtglykosyliertem Transferrin oder CDT, anschließendem Fusionieren der Milzzellen dieses Versuchstieres mit Myelomzellen, wobei antikörperproduzierende Hybridzellen entstehen und anschließendem Klonieren der Hybridzellen und Selektieren eines solchen Hybridzellklons, der einen Antikörper produziert, dessen Bindung nach den Ergebnissen einer Epitop-Kartierung im Bereich der folgenden Sequenzabschnitte (1) bis (4) eines CDT erfolgt:
(1) VVARSMGGKEDLIWELL und
(2) TTEDSIAKIMNGEADAMSLDGGF und
(3) SKLSMGSGLNLSEPN und
(4) YEKYLGEEYVKAV;
schließlich folgt die Gewinnung von Antikörpern aus dem solchermaßen selektierten Hybridzellklon nach einem dem Fachmann bekannten Verfahren.

Die vorstehend beschriebenen Herstellungsverfahren beinhalten die jedem Fachmann bekannte Hybridom-Technologie zur Herstellung monoklonaler Antikörper, wie sie erstmals im Jahre 1975 von Köhler und Milstein veröffentlicht und seitdem von zahlreichen Autoren modifiziert oder verbessert wurde. Obwohl diese Technologie häufig zur Herstellung von monoklonalen Antikörpern aus Mäusezellen verwendet wurden, gibt es auch Publikationen, welche die Herstellung monoklonaler Antikörper anderer Herkunft beschreiben. Darüber hinaus sind auch Verfahren zur Herstellung von Antikörperkonstrukten bekanntgeworden, beispielsweise humanisierter oder bi- oder oligospezifischer oder chimärer Antikörper, die selbstverständlich ebenso zur Herstellung erfindungsgemäßer Antikörper herangezogen werden können.

Die vorliegende Erfindung betrifft auch einen Immunoassay zum Nachweis von CDT in einer Probe; dabei wird ein vorstehend beschriebener erfindungsgemäßer Antikörper oder ein entsprechendes Antikörperfragment mit der Probe in Kontakt gebracht und anschließend die Bildung eines Immunkomplexes unter Beteiligung des CDT qualitativ oder quantitativ bestimmt.

Testkits zur Durchführung eines vorstehend genannten Immunoassays, enthaltend einen erfindungsgemäßen Antikörper oder ein erfindungsgemäßes Antikörperfragment sind ebenso Gegenstand der vorliegenden Erfindung.

Die vorliegende Erfindung wird außerdem durch die nachfolgenden Beispiele erläutert. Diese dienen ausschließlich der exemplarischen Beleuchtung einzelner Aspekte der vorliegenden Erfindung und sind keinesfalls als Einschränkung zu verstehen.

### Beispiele

### Beispiel 1: Herstellung von Anti-Human-Transferrin-Sepharose

Zur Affinitätsreinigung von Transferrin aus Humanseren (Normalseren und Alkoholiker-Seren) wurde ein Affinitätsträger durch Kopplung von 120 mg Anti-human Transferrin (Dade Behring Marburg GmbH, Marburg, Deutschland) an 0,8 g CNBraktivierte Sepharose CL-4B hergestellt.

120 mg Anti-human Transferrin werden gegen 0,1M NaHCO₃ -Lösung dialysiert. 0,8 g Sepharose CL-4B (Amersham Biosciences Europe GmbH, Freiburg, Deutschland) werden mit 0,1 M NaHCO₃-Lösung gewaschen und unter Kühlung mit 1,28 g Bromcyan gelöst in 5 ml Acetonitril versetzt. Die Suspension wird unter Rühren 15 Minuten bei pH 11 und 4°C gerührt. Anschließend wird die Suspension intensiv mit 0,1M NaHCO₃ -Lösung gewaschen. Die aktivierte Sepharose wird in 0,1 M NaHCO₃ -Lösung suspendiert und mit der vorbereiteten Antikörperlösung versetzt und 6 Stunden bei Raumtemperatur inkubiert. Die so hergestellte Anti-Human-Transferrin-Sepharose wird mit phosphatgepufferter Kochsalzlösung pH 7,2 gewaschen und bis zur Verwendung in phosphatgepufferter Kochsalzlösung pH 7,2 + 1g/L NaN₃ gelagert.

### Beispiel 2: Isolierung von Humantransferrin aus Humanserum (Normalserum und Alkoholiker-Serum)

Zur Affinitätsreinigung von Transferrin aus Humanserum wird die unter Beispiel 1 hergestellte Anti-Human-Transferrin-Sepharose in eine Glassäule gefüllt und mit 100ml phosphatgepufferter Kochsalzlösung pH 7,2 + 1 g/L NaN₃ gewaschen. 10ml Humanserum (Normalserum und Alkoholiker-Serum) werden mit einem Fluß von 0,5 ml/Minute auf die Säule aufgetragen und die nichtgebundenen Proteine durch Waschen der Säule mit 50 ml phosphatgepufferter Kochsalzlösung pH 7,2 + 1 g/L NaN₃ , 50 ml 1 M NaCI-Lösung und 50 ml Wasser entfernt. Das gebundene Transferrin wir mit 50 ml 0,5 M Glycin-Lösung, dessen pH-Wert mit Salzsäure auf pH 2,5 eingestellt wurde eluiert, sofort durch Zugabe von festem Tris(hydroxymethyl)-aminomethan neutralisiert und gegen phosphatgepufferter Kochsalzlösung pH 7,2 + 1g/L NaN₃ dialysiert.

### Beispiel 3: Nichtglykosyliertes Humantransferrin

### a) Rekombinantes nichtglykosyliertes Humantransferrin

Die Herstellung von rekombinanten nichtglykosyliertem Transferrin erfolgt mit Hilfe üblicher gentechnologischer und molekularbiologischer Methoden und wird in Mason et al. (1993) Biochemistry, 32: 5472-5479 beschrieben.

### b) Enzymatische Deglykosylierung von Humantransferrin

60 mg Humantransferrin (z.B. Fa. Calbiochem-Novabiochem GmbH, Bad Soden, Deutschland) werden in 8 ml phosphatgepufferter Kochsalzlösung pH 7,2 mit 10 mM/L ETDA und 1g/L (w/v) Natriumdecyl-sulfat (Fa. Fluka, Best.Nr: 71443) gelöst. Die so vorbereitete Transferrin-Lösung wird im Wasserbad auf 37°C erwärmt und 180 Einheiten (3 Einheiten / mg Transferrin) N Glycosidase F (Fa. Roche, Best.Nr: 1365193) zugegeben. Der Ansatz wird 17 Stunden im Wasserbad bei 37°C inkubiert. Die Vollständigkeit der Deglykosylierung wird mittels SDS-PAGE untersucht (Duan et al (1998) Applied Biochemistry and Biotechnology, 69: 217-224).

### Beispiel 4: Herstellung von monoklonalen Antikörpern gemäß dem Stand der Technik

Die Herstellung monoklonaler Antikörpern gemäß dem Stand der Technik erfolgte wie in der Patentschrift EP-0 605 627 B1 beschrieben durch Immunisierung mit transferrinspezifischen Peptidsequenzen P1 und P2. Es wurden die folgenden Hybride / monoklonalen Antikörper erhalten:

| **Antikörperbezeichnung:** | **Spezifität:** |
|---|---|
| 01-32/062 | anti-P1 |
| 00-177/012 | anti-P1 |
| 00-187/016 | anti-P2 |
| 00-187/027 | anti-P2 |

### Beispiel 5: Herstellung der erfindungsgemäßen, monoklonalen Antikörpern

### a) Immunisierung von Mäusen

BALB/c Mäuse wurden jeweils mit 20 µg nichtglykosyliertem Transferrin in komplettem Freund'schen Adjuvans intraperitoneal immunisiert. Nach 4 Wochen erfolgte ein Booster mit jeweils 20µg nichtglykosyliertem Transferrin in inkomplettem Freund'schen Adjuvans (Fa. ICN Biomedical GmbH, Eschwege, Deutschland) und nach 8 Wochen mit jeweils 20 µg nichtglykosyliertem Transferrin ohne Freund'sches Adjuvans. Die letzten 3 Tage vor der Fusion wurden die Mäuse intravenös mit jeweils 20 µg nichtglykosyliertem Transferrin geboostert.

### b) Fusion

Nach dem Töten der Mäuse durch CO₂-Inhalation wurden die Milzen entnommen und Einzelzellsuspensionen in serumfreiem Dulbeccos modifiziertem Eagle Medium (DMEM, Fa. CC Pro GmbH, Neustadt/W, Deutschland) hergestellt. Die Zellen wurden zentrifugiert (652 g) und 2 x in DMEM gewaschen. Anschließend wurde die Zellzahl mittels Trypanblau-Färbung bestimmt. Zu etwa 10⁸ Milzzellen wurden 2x10⁷ Myelomzellen (Sp2/0) gegeben. Nach dem Zentrifugieren (360 g) wurde der Überstand verworfen, 1 ml Polyethylenglycol-Lösung (PEG 4000, Fa. Merck Eurolab, Bruchsal, Deutschland; ca. 50%ig in DMEM) auf das Zellpellet gegeben und nach Resuspension 1 Minute bei 37°C inkubiert. Anschließend wurde tropfenweise ca. 10 ml DMEM zugegeben und 2 bis 4 Minuten bei Raumtemperatur inkubiert. Die fusionierten Zellen wurden abzentrifugiert (326 g) und das Pellet in DMEM + 20% FKS (fötales Kälberserum, Fa. Biowithaker Europe, Verviers, Belgien) + HAT-Lösung (Fa. CC Pro GmbH, Neustadt/W, Deutschland) resuspendiert und in 24 Well-Zellkulturplatten (Fa. Costar) abgefüllt. Die ungefähre Zellkonzentration pro Well betrug 5x10⁴ bis 5x10⁶ Zellen.

2 - 3 Wochen später wurden die entstandenen Zellkolonien (Hybride) entnommen und in neue Kulturplatten überführt.

### c) Bestimmung der Antikörperspezifität

Die Spezifität der in die Zellkultur abgegebenen Antikörper wurden in einem ersten Testschritt mit Hilfe von Immunisierungsantigen-beschichteten Mikrotitrationsplatten (Fa. Nunc, Typ B), Beschichtung 1 µg/ml ≈ 0,015 µg/Vertiefung, getestet.

In jede Vertiefung der Mikrotitrationsplatte wurden 100 µl Zellkulturüberstand (Verdünnung 1:2) pipettiert und 1 Stunde bei +15 bis +25°C inkubiert. Nach zweimaligen Waschen der Platte mit Waschlösung-POD (OSEW; Fa. Dade Behring, Marburg, Deutschland) wurden in jede Vertiefung 100 µl anti-Maus IgG/F(ab)₂-POD-Konjugat (Fa. Dade Behring, Marburg, Deutschland) eingefüllt und 1 Stunde bei +15 bis +25°C inkubiert. Nach weiterem zweimaligen Waschen der Platte wurde in jede Vertiefung 100 µl Chromogen TMB-Lösung (Fa. Dade Behring, Marburg, Deutschland) eingefüllt und weitere 30 Minuten bei +15 bis +25°C inkubiert. Nach der Inkubation wurde in jede Vertiefung 100 µl Stopplösung POD (Fa. Dade Behring, Marburg. Deutschland) eingefüllt und die Mikrotitrationsplatte am BEP II (Behring-ELISA-Prozessor II, Fa. Dade Behring, Marburg, Deutschland) bei 450 nm ausgewertet.

In einem zweiten Testschritt wurden die Hybride wie oben beschrieben überprüft mit Hilfe von Mikrotitrationsplatten (Fa. Nunc, Typ B), die mit Humantransferrin (beispielsweise von Fa. Calbiochem-Novabiochem GmbH, Bad Soden, Deutschland) beschichtet waren. Beschichtung 1 µg/ml ≈ 0,015 µg/Vertiefung.

Die Ergebnisse sind in Tabelle 1 aufgelistet.

**Tabelle 1: Bestimmung der Antikörperspezifität durch Auswertung der Mikrotitrationsplatten am BEP II (Behring-ELISA-Prozessor II) bei 450 nm.**

| | Extinktion bei 450 nm | |
|---|---|---|
| Hybridbezeichnung | Nichtglykosyliertes Humantransferrin | Humantransferrin |
| 98-22/026(569) | > 2,5 | Negativ |
| 98-23/07 (45) | > 2,5 | Negativ |
| 98-22/0104 (572) | 1,739 | Negativ |
| 98-84/011 (1) | > 2,5 | Negativ |
| 01-102/01 (113) | > 2,5 | Negativ |

| | | |
|---|---|---|
| Legende: negativ = Extinktion _{(450 nm)} < 0,1 OD; bei Verdünnung der untersuchten Hybride keine Abstufung des Signals | | |

### d) Klonierung

Einzelne Zellen von Hybriden, die die gewünschten Antikörper produzieren (Bindung an nichtglykosyliertes human Transferrin nicht jedoch an human Transferrin, wurden mit einem Mikromanipulator (Fa. Leitz, Wetzlar, Deutschland) kloniert. Die so erhaltene Klone 98-84/011 und 01-102/01 wurden am 16.04.2002 bei der DSMZ Deutsche Sammlung von Mikroorganismen und Zellkulturen GmbH, Mascheroder Weg 1 b, Braunschweig, Deutschland, unter der Eingangsnummer DSM ACC2540 (98-84/011) und DSM ACC2541 (01-102/01) hinterlegt.

### e) Bestimmung der Antikörpersubklasse

Die Subklasse der Antikörpers 98-84/011 und 01-102/01 wurde mittels IsoStrip™-Mouse Monoclonal Antibody Isotyping Kit- der Fa. Boehringer Mannheim, Deutschland als IgG₁ für 98-84/011 und 01-102/01 bestimmt.

### f) Produktion der Antikörper

Für die Produktion größerer Mengen Antikörper werden die entsprechenden Zellklone in Rollerflaschen (Fa. Corning Costar Deutschland, Bodenheim) überführt, und bis zum gewünschten Endvolumen bei +37°C expandiert. Danach wird die Rollerkultur-Suspension zur Entfernung der Zellen über 0,22 µm filtriert. Die jetzt zellfreie Antikörperlösung wird über Ultrafilter (Trenngrenze 30.000 Dalton) ankonzentriert und anschließend aufgereinigt.

### g) Reinigung der Antikörper

Die erhaltene Antikörperlösung wird gegen 0,14 M Phosphatpuffer pH 8,6 umgepuffert und auf ein mit rProtein A Sepharose Fast Flow (Fa. Amersham Pharmacia) gefüllte Chromatographiesäule aufgetragen (pro 10 mg zu reinigender Antikörper werden 1 ml rProtein A Sepharose Fast Flow eingesetzt). Alle nicht gebundenen Komponenten werden durch Waschen der Säule mit 0,14 M Phosphatpuffer pH 8,6 entfernt. Der gebundene Antikörper wird mit 0,1 M Zitronensäure pH 3,0 von der Säule eluiert und gegen 0,05 M Natriumacetat + 0,5 M NaCl + 0,05 M Tris + 0,01% Natriumazid pH 7,0 dialysiert.

### Beispiel 6: Bestimmung der Spezifität der Antikörper für festphasengebundene Antigene

Die Spezifität der gewonnenen Antikörper wurde mit Hilfe von a) mit nichtglykosyliertem Transferrin beschichteten Mikrotitrationsplatten (Fa. Nunc, Typ B), Beschichtung 1 µg/ml ≈ 0,015 µg/Vertiefung, b) mit Humantransferrin beschichteten Mikrotitrationsplatten (Fa. Nunc, Typ B), Beschichtung 1 µg/ml ≈ 0,015 µg/Vertiefung, c) mit Peptid P1 beschichteten Mikrotitrationsplatten (Fa. Nunc, Typ B), Beschichtung 3 µg/ml ≈ 0,045 µg/Vertiefung und d) mit Peptid P2 beschichteten Mikrotitrationsplatten (Fa. Nunc, Typ B), Beschichtung 3 µg/ml ≈ 0,045 µg/Vertiefung, getestet.

In jede Vertiefung der Mikrotitrationsplatte wurden 100 µl monoklonaler Antikörper (1 µg/ml) pipettiert und 1 Stunde bei +15 bis +25°C inkubiert. Nach zweimaligem Waschen der Platte mit Waschlösung-POD (OSEW; Fa. Dade Behring, Marburg, Deutschland) wurden in jede Vertiefung 100 µl anti-Maus IgG/F(ab)₂-POD-Konjugat (Fa. Dade Behring, Marburg, Deutschland) eingefüllt und 1 Stunde bei +15 bis +25°C inkubiert. Nach weiterem zweimaligen Waschen der Platte wurden in jede Vertiefung 100 µl Chromogen TMB-Lösung (Fa. Dade Behring, Marburg, Deutschland) eingefüllt und weitere 30 Minuten bei +15 bis +25°C inkubiert. Nach der Inkubation wurden in jede Vertiefung 100 µl Stopplösung POD (Fa. Dade Behring, Marburg. Deutschland) eingefüllt und die Mikrotitrationsplatte am BEP II (Fa. Dade Behring, Marburg, Deutschland) bei 450 nm ausgewertet.

Die Ergebnisse sind in Tabelle 2 aufgelistet.

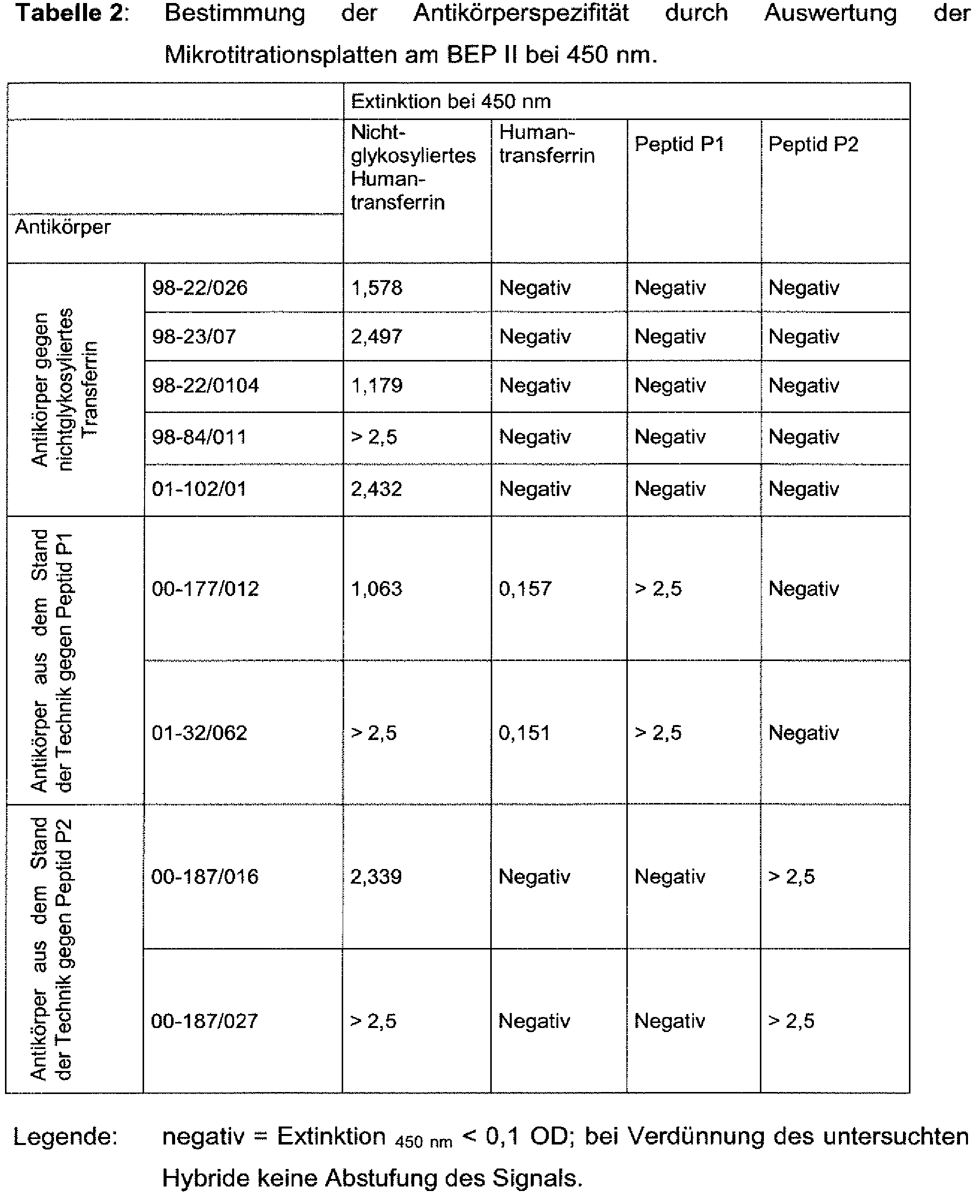

Die mit nichtglykosyliertem Transferrin induzierten Antikörper zeigen nur eine Reaktion mit nichtglykosyliertem Transferrin, wobei die Antikörper aus dem Stand der Technik eine Reaktion mit dem jeweiligen Peptid und dem auf der Festphase gebundenen nichtglykosylierten Transferrin zeigen.

### Beispiel 7: Bestimmung der Spezifität der Antikörper für Antigene in Lösung

### a) Mikrotitrationsplatten (Fa. Nunc, Typ B), wurden mit den zu vergleichenden monoklonalen Antikörpern beschichtet. Beschichtungskonzentration 1 µg/ml ≈ 0,015 µg/Vertiefung.

In die Vertiefungen der Mikrotitrationsplatte wurden 100 µl einer geometrischen Verdünnungsreihe beginnend bei 200 µg/ml von a) Humantransferrin, b) enzymatisch deglykosyliertem Humantransferrin, c) Humantransferrin aus Normalserum und d) Humantransferrin aus Alkoholikerserum pipettiert und 1 Stunde bei +15 bis +25°C inkubiert. Nach zweimaligem Waschen der Platte mit Waschlösung-POD (OSEW; Fa. Dade Behring, Marburg, Deutschland) wurden in jede Vertiefung 100 µl Anti-Human-Transferrin-POD-Konjugat (Fa. Dade Behring, Marburg, Deutschland) eingefüllt und 1 Stunde bei +15 bis +25°C inkubiert. Nach weiterem zweimaligen Waschen der Platte wurden in jede Vertiefung 100 µl Chromogen TMB-Lösung (Fa. Dade Behring, Marburg, Deutschland) eingefüllt und weitere 30 Minuten bei +15 bis +25°C inkubiert. Nach der Inkubation wurden in jede Vertiefung 100 µl Stopplösung POD (Fa. Dade Behring, Marburg. Deutschland) eingefüllt und die Mikrotitrationsplatte am BEP II (Fa. Dade Behring, Marburg, Deutschland) bei 450 nm ausgewertet.

Die Ergebnisse sind in Tabelle 3.1 und 3.2 aufgelistet.

**Tabelle 3.1: Bestimmung der Reaktivität durch Auswertung der Mikrotitrationsplatten am BEP II bei 450 nm.**

| | | Extinktion bei 450 nm | | | | | | | | | | | | | |
|---|---|---|---|---|---|---|---|---|---|---|---|---|---|---|---|
| | | Antikörper gegen nichtglykosyliertes Transferrin | | | Antikörper gemäß dem Stand der Technik | | | | | Antikörper gegen nichtglykosyliertes Transferrin | | | Antikörper gemäß dem Stand der Technik | | |
| Antigen | Konz [µg/ml] | 98-23/07 | 98-84/011 | 01-102/01 | 03-32/062 | 00-187/016 | 00-187/027 | Antigen | Konz [µg/ml] | 98-23/07 | 98-84/011 | 01-102/01 | 01-32/062 | 00-187/016 | 00-187/027 |
| Human-transferrin | 200 | 1,790 | 2,5 | 0,137 | Negativ | 0,508 | 0,553 | Nichtglykosyliertes Human-transferrin | 200 | 2,500 | 2,500 | 1,773 | 0,388 | 2,500 | 2,500 |
| | 100 | 0,664 | 2,5 | Negativ | Negativ | 0,230 | 0,291 | | 100 | 2,500 | 2,500 | 1,582 | 0,262 | 2,193 | 2,500 |
| | 50 | 0,541 | 2,5 | Negativ | Negativ | 0,123 | 0,170 | | 50 | 2,500 | 2,500 | 1,570 | 0,160 | 1,406 | 2,133 |
| | 25 | 0,491 | 2,5 | Negativ | Negativ | Negativ | Negativ | | 25 | 2,500 | 2,500 | 1,601 | 0,104 | 0,714 | 1,134 |
| | 12,5 | 0,320 | 2,5 | Negativ | Negativ | Negativ | Negativ | | 12,5 | 2,500 | 2,500 | 1,274 | Negativ | 0,442 | 0,588 |
| | 6,25 | 0,158 | 2,5 | Negativ | Negativ | Negativ | Negativ | | 6,25 | 2,500 | 2,500 | 1,238 | Negativ | 0,233 | 0,320 |
| | 3,125 | Negativ | 1,880 | Negativ | Negativ | Negativ | Negativ | | 3,125 | 2,500 | 2,500 | 1,230 | Negativ | 0,133 | 0,183 |
| | 1,56 | Negativ | 0,604 | Negativ | Negativ | Negativ | Negativ | | 1,56 | 2,500 | 2,500 | 0,880 | Negativ | Negativ | Negativ |
| | 0,781 | Negativ | 0,407 | Negativ | Negativ | Negativ | Negativ | | 0,781 | 2,500 | 2,500 | 0,890 | Negativ | Negativ | Negativ |
| | 0,391 | Negativ | 0,284 | Negativ | Negativ | Negativ | Negativ | | 0,391 | 2,500 | 2,500 | 0,722 | Negativ | Negativ | Negativ |
| | 0,195 | Negativ | 0,169 | Negativ | Negativ | Negativ | Negativ | | 0,195 | 2,500 | 2,500 | 0,436 | Negativ | Negativ | Negativ |

| | | | | | | | | | | | | | | | |
|---|---|---|---|---|---|---|---|---|---|---|---|---|---|---|---|
| negativ : Extinktion (450 nm) < 0,1 OD positiv: Extinktion _{(450 nm)} ≥ 0,1 OD | | | | | | | | | | | | | | | |

**Tabelle 3.2: Bestimmung der Reaktivität durch Auswertung der Mikrotitrationsplatten am BEP II bei 450 nm.**

| | | Extinktion bei 450 nm | | | | | | | | | | | | | |
|---|---|---|---|---|---|---|---|---|---|---|---|---|---|---|---|
| | | Antikörper gegen nichtglykosyliertes Transferrin | | | Antikörper gemäß dem Stand der Technik | | | | | Antikörper gegen nichtglykosyliertes Transferrin | | | Antikörper gemäß dem Stand der Technik | | |
| Antigen | Konz [µg/ml] | 98-23/07 | 98-84/011 | 01-102/01 | 01-32/062 | 00-187/016 | 00-187/027 | Antigen | Konz [µg/ml] | 98-23/07 | 98-84/011 | 01-102/01 | 01-32/062 | 00-187/016 | 00-187/027 |
| Human-transferrin aus Normalserum | 200 | 0,309 | 2,5 | 0,188 | Negativ | 0,142 | 0,192 | Human-Transferrin aus Alkoholiker-serum | 200 | 0,508 | 2,5 | Negativ | Negativ | 0,118 | 0,133 |
| | 100 | 0,229 | 2,5 | 0,116 | Negativ | Negativ | 0,158 | | 100 | 0,660 | 2,5 | Negativ | Negativ | Negativ | Negativ |
| | 50 | 0,177 | 2,5 | Negativ | Negativ | Negativ | 0,111 | | 50 | 0,306 | 2,5 | Negativ | Negativ | Negativ | Negativ |
| | 25 | 0,141 | 2,5 | Negativ | Negativ | Negativ | Negativ | | 25 | 0,252 | 2,5 | Negativ | Negativ | Negativ | Negativ |
| | 12,5 | 0,100 | 2,5 | Negativ | Negativ | Negativ | Negativ | | 12,5 | 0,181 | 2,5 | Negativ | Negativ | Negativ | Negativ |
| | 6,25 | Negativ | 2,5 | Negativ | Negativ | Negativ | Negativ | | 6,25 | 0,101 | 2,5 | Negativ | Negativ | Negativ | Negativ |
| | 3,125 | Negativ | 2,5 | Negativ | Negativ | Negativ | Negativ | | 3,125 | Negativ | 2,5 | Negativ | Negativ | Negativ | Negativ |
| | 1,56 | Negativ | 1,234 | Negativ | Negativ | Negativ | Negativ | | 1,56 | Negativ | 2,5 | Negativ | Negativ | Negativ | Negativ |
| | 0,781 | Negativ | 0,745 | Negativ | Negativ | Negativ | Negativ | | 0,781 | Negativ | 2,5 | Negativ | Negativ | Negativ | Negativ |
| | 0,391 | Negativ | 0,450 | Negativ | Negativ | Negativ | Negativ | | 0,391 | Negativ | 1,676 | Negativ | Negativ | Negativ | Negativ |
| | 0,195 | Negativ | 0,245 | Negativ | Negativ | Negativ | Negativ | | 0,195 | Negativ | 0,920 | Negativ | Negativ | Negativ | Negativ |

| | | | | | | | | | | | | | | | |
|---|---|---|---|---|---|---|---|---|---|---|---|---|---|---|---|
| negativ: Extinktion _{(450 nm)} < 0,1 OD positiv: Extinktion _{(450 nm)} ≥ 0,1 OD | | | | | | | | | | | | | | | |

### b) Mikrotitrationsplatten (Fa. Nunc, Typ B), wurden mit den monoklonalen Antikörpern beschichtet. Beschichtungskonzentration 3 µg/ml ≈ 0,045 µg/Vertiefung.

In die Vertiefungen der Mikrotitrationsplatte wurden 100 µl einer geometrischen Verdünnungsreihe beginnend bei einer 1:10 Verdünnung von a) Normalserum und b) Alkoholikerserum pipettiert und 1 Stunde bei +15 bis +25°C inkubiert. Nach zweimaligen Waschen der Platte mit Waschlösung-POD (OSEW; Fa. Dade Behring, Marburg, Deutschland) wurden in jede Vertiefung 100 µl Anti-Human-Transferrin-POD-Konjugat (Fa. Dade Behring, Marburg, Deutschland) eingefüllt und 1 Stunde bei +15 bis +25°C inkubiert. Nach weiterem zweimaligen Waschen der Platte wurde in jede Vertiefung 100 µl Chromogen TMB-Lösung (Fa. Dade Behring, Marburg, Deutschland) eingefüllt und weitere 30 Minuten bei +15 bis +25°C inkubiert. Nach der Inkubation wurde in jede Vertiefung 100 µl Stopplösung POD (Fa. Dade Behring, Marburg. Deutschland) eingefüllt und die Mikrotitrationsplatte am BEP II (Fa. Dade Behring, Marburg, Deutschland) bei 450 nm ausgewertet.

Die Ergebnisse sind in Tabelle 4 aufgelistet.

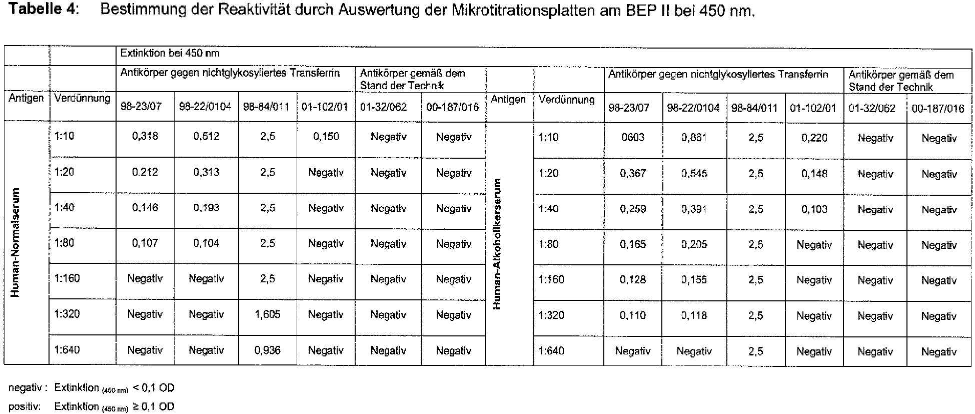

Die gegen nichtglykosyliertes Transferrin gerichteten Antikörper ermöglichen eine deutliche Differenzierung zwischen Transferrin (im Normalserum) und CDT (im Alkoholiker-Serum), wobei die Antikörper aus dem Stand der Technik mit beiden Seren keine Reaktion zeigen.

### Beispiel 8: Epitop-Kartierung

Scans überlappender Peptide, die aus der Sequenz des humanen Transferrins abgeleitet wurden (13-mere Peptide, 11 Aminosäuren überlappend), wurden mit Hilfe der SPOT-Synthese-Technologie hergestellt. Die Verfahren sind beschrieben in: Wenschuh, H. et al. (2000) Coherent membrane supports for parallel microsynthesis and screening of bioactive peptides, Biopolymers (Peptide Science), 55:188-206. Die Peptide wurden C-terminal an einen Celluloseträger gekoppelt und tragen am N-Terminus einen Reaktivitäts-tag. Nach Abspaltung der Peptide von ausgestanzten SPOTs (96-well Mikrotitrationsplatten) wurden sie auf aktivierte Glas-chips gekoppelt. Das Inkubationsprotokoll für diese Glas-chips lautet wie folgt:

### Monoklonale Antikörper gemäß dem Stand der Technik

- Äquilibrierung in TBS-Puffer, pH 8.0
- 2 h Blockierungspuffer, pH 8.0
- 2 h Antikörperinkubation (3 µg/ml) in Blockierungspuffer, pH 8.0
- Waschen mit TBS (0.05% Tween20)
- 2 h Inkubation mit anti-Maus-IgG-POD in Blockierungspuffer, pH 8.0
- 3 x 5 min Waschen mit TBS (0.05% Tween20)
- Detektion mittels Chemolumineszenz (Lumi-Imager), Roche Diagnostics)

### Erfindungsgemäßer Antikörper 98-84/011

- Äquilibrierung in TBS-Puffer, pH 8.0
- 2 h Blockierungspuffer, pH 8.0
- 2 h Antikörperinkubation (3 µg/ml) in Blockierungspuffer, pH 8.0
- 3 x 5 min Waschen mit TBS (0.05% Tween20)
- Detektion mittels Chemolumineszenz (Lumi-Imager), Roche Diagnostics)

Der erfindungsgemäße Antikörper wurde direkt mit Peroxidase markiert. Die Methode ist in der Literatur beschrieben: Wilson, M. B. and Nakane, P. K. (1978) Recent developments in the periodate method of conjugating harseradish peroxidase (HRPO) to antibodies, In: Immunofluorescence and Related Staining Techniques (Eds.: Knapp, W.; Holubar, K.; Wick, G.) pp. 215-224.

Nach Auswertung der Untersuchung ergeben sich für die Antikörper gemäß dem Stand der Technik die folgenden bindenden Peptide:
Antikörper gemäß dem Stand der Technik gegen Peptid 1

| | |
|---|---|
| 1. | VLAENY**NKSDNCE** |
| 2. | AENY**NKSDNCE**DT |
| 3. | NY**NKSDNCE**DTPE |
| 4. | **NKSDNCE**DTPEAG |

Antikörper aus dem Stand der Technik gegen Peptid 2

| | |
|---|---|
| 1. | VHKILRQQ**QHLFG** |
| 2. | KILRQQ**QHLFG**SN |
| 3. | LRQQ**QHLFG**SNVT |
| 4. | QQ**QHLFG**SNVTDC |
| 5. | **QHLFG**SNVTDCSG |

Die erkannten Sequenzen sind identisch mit den zur Immunisierung eingesetzten Peptiden.

Der erfindungsgemäße Antikörper 98-84/011 reagiert mit vier dominanten Sequenzabschnitten:

| | |
|---|---|
| 1. | VVAR**SMGGKEDLI** |
| 2. | AR**SMGGKEDLI**WE |
| 3. | **SMGGKEDLI**WELL |
| | |
| 4. | TTEDSIAKIM**NGE** |
| 5. | SIAKIM**NGE**ADAM |
| 6. | AKIM**NGE**ADAMSL |
| 7. | TM**NGE**ADAMSLDG |
| 8. | **NGE**ADAMSLDGGF |
| | |
| 9. | SK**LSMGSGLNLSE** |
| 10. | **LSMGSGLNLSE**PN |
| | |
| 11. | YEKYLGEEYVKAV |

Der Bereich 1. - 3. befindet sich in der N-terminalen Domäne des Transferrins , während die Bereiche 4. - 8., 9. - 10. und 11. in der C-terminalen Domäne liegen und ein diskontinuierliches Epitop darstellen.

## Patentansprüche

1. Monoklonaler Antikörper, hergestellt durch Immunisieren eines geeigneten nicht-menschlichen Versuchstieres mit nichtglykosyliertem Transferrin oder Carbohydrate Deficient Transferrin (CDT) und die Etablierung von Hybridomazellen, der in wäßriger Lösung selektiv an CDT bindet, ohne daß dieses an eine feste Phase gebunden zu sein braucht und dessen Bindung im Bereich der folgenden Sequenzabschnitte (1) bis (4) des CDT erfolgt:
(1) VVARSMGGKEDLIWELL und
(2) TTEDSIAKIMNGEADAMSLDGGF und
(3) SKLSMGSGLNLSEPN und
(4) YEKYLGEEYVKAV.

2. Monoklonaler Antikörper gemäß Anspruch 1, **dadurch gekennzeichnet, daß** er von der Zellkultur mit der Hinterlegungsnummer DSM ACC2540 produziert wird.

3. Antigenbindendes Fragment eines Antikörpers gemäß einem der vorhergehenden Ansprüche 1 oder 2, welches dieselbe Antigenspezifität aufweist wie der in einem dieser Ansprüche definierte Antikörper.

4. Verfahren zur Herstellung des Antikörpers gemäß Anspruch 1 durch Immunisieren eines geeigneten nicht-menschlichen Versuchstieres mit nichtglykosyliertem Transferrin oder CDT, Fusionieren der Milzzellen dieses Versuchstieres mit Myelomzellen, wobei antikörperproduzierende Hybridzellen entstehen, Klonieren der Hybridzellen und Selektieren eines solchen Hybridzellklons, der einen Antikörper produziert, der in wäßriger Lösung selektiv an CDT bindet und dessen Bindung im Bereich der folgenden Sequenzabschnitte (1) bis (4) des CDT erfolgt:
(1) VVARSMGGKEDLIWELL und
(2) TTEDSIAKIMNGEADAMSLDGGF und
(3) SKLSMGSGLNLSEPN und
(4) YEKYLGEEYVKAV.

5. Immunoassay zum Nachweis von CDT in einer Probe, **dadurch gekennzeichnet, daß** ein Antikörper gemäß einem der Ansprüche 1 oder 2 oder das antigenbindende Fragment gemäß Anspruch 3 mit der Probe in Kontakt gebracht und die Bildung eines Immunkomplexes unter Beteiligung des CDT qualitativ oder quantitativ bestimmt wird.

6. Testkit zur Durchführung eines Immunoassays gemäß Anspruch 5 enthaltend einen Antikörper gemäß einem der Ansprüche 1 oder 2 oder das antigenbindende Fragment gemäß Anspruch 3.

## Claims

1. A monoclonal antibody prepared by immunizing a suitable non-human experimental animal with unglycosylated transferrin or carbohydrate deficient transferrin (CDT) and by establishing hybridoma cells, which binds selectively in aqueous solution to CDT without the latter needing to be bound to a solid phase and whose binding takes place in the region of the following segments (1) to (4) of the CDT sequence:
(1) VVARSMGGKEDLIWELL and
(2) TTEDSIAKIMNGEADAMSLDGGF and
(3) SKLSMGSGLNLSEPN and
(4) YEKYLGEEYVKAV.

2. A monoclonal antibody as claimed in claim 1, which is produced by the cell culture having the deposition number DSM ACC2540.

3. An antigen-binding fragment of an antibody as claimed in either of the preceding claims 1 and 2 which has the same antigen specificity as the antibody defined in any of these claims.

4. A process for preparing the antibody as claimed in claim 1 by immunizing a suitable non-human experimental animal with unglycosylated transferrin or CDT, fusing the spleen cells of this experimental animal to myeloma cells, resulting in antibody-producing hybrid cells, cloning the hybrid cells and selecting a hybrid cell clone which produces an antibody which selectively binds to CDT in aqueous solution and whose binding takes place in the region of the following segments (1) to (4) of the CDT sequence:
(1) VVARSMGGKEDLIWELL and
(2) TTEDSIAKIMNGEADAMSLDGGF and
(3) SKLSMGSGLNLSEPN and
(4) YEKYLGEEYVKAV.

5. An immunoassay for detecting CDT in a sample, which comprises bringing an antibody as claimed in either of claims 1 and 2 or the antigen-binding fragment as claimed in claim 3 into contact with the sample, and determining qualitatively or quantitatively the formation of an immune complex involving CDT.

6. A test kit for carrying out an Immunoassay as claimed in claim 5 comprising an antibody as claimed in either of claims 1 and 2 or the antigen-binding fragment as claimed in claim 3.

## Revendications

1. Anticorps monoclonal, produit par immunisation d'un animal d'expérience non-humain approprié par de la transferrine non glycosylée ou de la transferrine déficiente en carbohydrate (CDT) et l'établissement de cellules d'hybridome, qui, en solution aqueuse, se fixent sélectivement à la CDT sans que celle-ci ait besoin d'être fixée à une phase solide et dont la fixation s'effectue dans la région des segments (1) à (4) de séquence suivants de la CDT :
(1) VVARSMGGKEDLIWELL et
(2) TTEDSIAKIMNGEADAMSLDGGF et
(3) SKLSMGSGLNLSEPN et
(4) YEKYLGEEYVKAV.

2. Anticorps monoclonal suivant la revendication 1, **caractérisé en ce qu'**il est produit par la culture cellulaire, ayant le numéro de dépôt DSM ACC2540.

3. Fragment d'un anticorps fixant un antigène suivant l'une des revendications précédentes 1 ou 2, qui a la même spécificité antigénique que l'anticorps défini dans l'une de ces revendications.

4. Procédé de production de l'anticorps suivant la revendication 1, par immunisation d'un animal d'expérience non-humain approprié par de la transferrine non glycosylée ou par de la CDT, fusion des splénocytes de cet animal d'expérience avec des cellules de myélome en créant des cellules hybrides produisant des anticorps, clonage des cellules hybrides et sélection d'un clone des cellules hybrides de ce genre, qui produit un anticorps, qui, en solution aqueuse, se fixe sélectivement sur de la CDT et dont la fixation s'effectue dans la région des segments (1) à (4) de séquence suivants de la CDT :
(1) VVARSMGGKEDLIWELL et
(2) TTEDSIAKIMNGEADAMSLDGGF et
(3) SKLSMGSGLNLSEPN et
(4) YEKYLGEEYVKAV.

5. Immunodosage de détection de la CDT dans un échantillon, **caractérisé en ce qu'**on met un anticorps suivant l'une des revendications 1 ou 2 ou le fragment fixant un antigène suivant la revendication 3 en contact avec l'échantillon et on détermine qualitativement ou quantitativement la formation d'un immunocomplexe avec participation de la CDT.

6. Groupe de test pour effectuer un immunodosage suivant la revendication 5, contenant un anticorps suivant l'une des revendications 1 ou 2 ou le fragment fixant un antigène suivant la revendication 3.
